# EUROPEAN PATENT SPECIFICATION

(11) **EP 1 330 244 B1**
(45) Date of publication and mention of the grant of the patent: **17.12.2008**
(21) Application number: 01979747.1
(22) Date of filing: 11.10.2001
(51) Int. Cl.: A61K 31/165, A61K 9/10, A61K 9/48, A61K 47/14, A61K 47/26

(54) **COMPOSITIONS COMPRISING MODAFINIL COMPOUNDS**
ARZNEIZUSAMMENSETZUNGEN ENTHALTEND MODAFINILVERBINDUNGEN
COMPOSITIONS COMPRENANT DES COMPOSES DE MODAFINIL

(30) Priority: 11.10.2000 US 239490 P; 10.10.2001 US 640824
(43) Date of publication of application: 30.07.2003
(73) Proprietor: CEPHALON, INC., Frazer, PA 19355 (US)
(72) Inventor: JACOBS, Martin, J., West Chester, PA 19380 (US); MCINTYRE, Bradley, T., Thorndale, PA 19372 (US); PATEL, Piyush, R., Wallingford, PA 19086 (US)
(74) Representative: Hallybone, Huw George
(86) International application number: PCT/US2001/031904
(87) International publication number: WO 2002/030414

(56) References cited:
- WO-A-94/21371
- WO-A-96/11001
- WO-A-99/25329

## Description

### FIELD OF THE INVENTION

The invention relates to particle-forming compositions comprising a modafinil compound. The invention is also directed to compositions of suspended particles which are formed when the particle-forming compositions are contacted with an aqueous medium. The invention is further directed to methods of preparation of the compositions, and the use of the compositions in the manufacture of medicaments for the treatment of diseases to a subject in need thereof.

### BACKGROUND OF THE INVENTION

Modafinil (C₁₅H₁₅NO₂S), is 2-(benzhydryl-sulfinyl)acetamide, and is also known as 2-[(diphenylmethyl) sulfinyl] acetamide.

Modafinil has been described as presenting a "neuropsychopharmacological spectrum characterized by the presence of excitation with hyperactivity and of hypermotility; and by the absence of stereotypy (except in high doses) and of potentialization of the effects of apomorphine and amphetamine" (U.S. Patent 4,177,290; hereinafter the "290 patent. A single administration of modafinil results in increased locomotor activity in mice and increased nocturnal activity, in monkeys (Duteil et al., Eur. J. Pharmacol. 180:49 (1990)). Modafinil has been successfully tested in humans for treatment of idiopathic hypersomnia and narcolepsy (Bastuji et al., Prog. Neuro-Psych. Biol. Psych. 12:695 (1988)).

Other uses of modafinil have been presented. U.S. Patent 5,1.80,745, discloses the use of modafinil for providing a neuroprotective effect in humans, and in particular for the treatment of Parkinson's disease. The levorotatory form of modafinil, i.e., (-)benzhydrylsulfinyl-acetamide, may have potential benefit for treatment of depression, hypersomnia and Alzheimer's disease (U.S. Patent 4,927,855 ). European Published Application 547952 (published June 23, 1993) discloses the use of modafinil as an anti-ischemic agent. European Published Application 594507 (published April 27, 1994) discloses the use of modafinil to treat urinary incontinence.

Preparations of modafinil having a defined solid particle size have been described in U.S. Pat. No. 5,618,845 and preparations of a levorotatory isomer of modafinil was described in U.S. Patent No. 4,927,855. Heterocyclic derivatives of modafinil are disclosed in U.S. Patent Application No. 60/204,789.

Modafinil has been approved for use in humans in 100 mg and 200 mg solid unit dose forms in the U.S. It is also desirable to formulate modafinil in liquid compositions. It has been observed that modafinil has very poor water and lipid solubility and it is therefore difficult to solubilize modafinil in pharmaceutically-acceptable compositions. Conventional solid and liquid formulations that include modafinil are described in the '290 patent. Liquid suspensions or emulsions of modafinil were mentioned in U.S. Pat. No. 5,618,845. A suspension of modafinil was reported in U.S. Pat. No. 5,180,745. An aqueous cyclodextrin solution of modafinil was described in Rambert, F.A., et al. Neuropsychopharmacology, 10(3S), Part 2 (May 1994). Modafinil freeze-dried particles are also disclosed in WO 9421371 .

A technique recently developed to formulate liquid pharmaceutical compositions for agents that display very low water solubility involves a self-emulsifying drug delivery system, known as "SEDDS". These drug delivery systems are isotropic mixtures of lipids or lipid-soluble compounds and a surfactant that rapidly form thermodynamically stable microparticles upon contact with water. See, e.g., Shah et al., International Journal of Pharmaceutics (Netherlands), 106:15-23, (1994).

Respite the low lipid solubility of modafinil, it has been discovered that modafinil can be formulated to produce-particle-forming compositions, wherein the compositions are capable of forming particles comprising a modafinil compound upon contact with water. These compositions have been found to effectively solubilize modafinil in an aqueous component and to provide for effective bioavailable delivery of modafinil to u subject in need thereof.

### SUMMARY OF THE INVENTION

Accordingly, one object of the present invention is to provide particle-forming compositions comprising a modafinil compound. Particularly, the particle-forming compositions of the present invention are non-aqueous and comprise an organic solvent and an amount of at least one surfactant sufficient to allow for the formation of a composition of particles upon contact of the particle-forming composition with an aqueous medium.

It is another object of the invention to provide for compositions of particles in an aqueous medium, wherein the particles comprise a modafinil compound. Preferably the compositions of particles comprise a stable suspension, wherein the suspended particles comprise a modafinil compound.

Preferably, both the particle-forming compositions and the compositions of particles, wherein the particles comprise a modafinil compound, are pharmaceutically acceptable compositions and allow for bioavailable delivery of a modafinil compound upon oral administration to a subject in need thereof.

It is another object of the invention to provide a method of forming a composition of particles in an aqueous medium which comprises contacting the particle-forming compositions comprising a modafinil compound with an aqueous medium.

It is another object of the invention to provide the use of either of the compositions of the invention in the manufacture of a medicament for treating a disease or disorder in a subject.

These and other objects, which will become apparent during the following detailed description, have been achieved by the inventors' discovery that despite its poor aqueous and lipid solubility, a modafinil compound can be formulated to provide effective bioavailability upon administration to a subject in need thereof.

### DETAILED DESCRIPTION OF THE INVENTION

Thus, in a first embodiment, the present invention provides a particle-forming composition comprising a modafinil compound, at least one surfactant, and an organic solvent, characterized in that the composition spontaneously forms a stable suspension of non-crystalline particles comprising the modafinil compound when contacted with an aqueous medium, wherein the non-crystalline particles have a diameter of 1 to 1,000 nm. Preferably the particle-forming composition comprises modafinil.

In a second embodiment, the present invention provides an aqueous composition of non-crystalline particles comprising at least one surfactant, an organic solvent, and a modafinil compound, wherein the non-crystalline particles have a diameter of 1 to 1,000 nm, and wherein the composition is liquid, homogeneous, stable; translucent, and optically isotropic. Preferably the particles comprise modafinil.

In certain preferred embodiments, the compositions are pharmaceutically acceptable.

As used herein, "the compositions" refers collectively to the particle-forming compositions and the compositions of particles wherein the particles comprise a modafinil compound.

As used herein, a "non-aqueous" compositions refers to a composition that contains from 0-10 % water by weight.

As used herein, "aqueous medium" refers to any medium comprised of greater than 10°C water.

As used herein, a "particle-forming composition" refers to a composition that is capable of forming particles upon contact with an aqueous medium. Preferably the particle-forming composition is a liquid or solid solution.

As used herein, "particle" or "particles" refers to substantially non-crystalline structures, preferably an aggregation of molecules in a discrete non-crystalline structure, such as a micelle, microsphere, droplet, colloid, or globule. Preferably the particles comprise a modafinil compound, and more preferably, comprise modafinil,

As used herein, "composition of particles" refers to a composition comprising a particle wherein the particle comprises a modafinil compound.

As used herein, "stable suspension" refers to a mixture of particles that remain intact and dispersed in a liquid medium such that the suspension can be stored and administered in a pharmaceutically acceptable manner.

As used herein, "a modafinil compound" or "modafinil compound" refers to modafinil, its racemic mixtures, individual isomers, acid addition salts, such as a metabolic acid of modafinil, benzhydrylsulfinylacetic acids, and its sulfone forms, hydroxylated forms and polymorphic forms . In certain preferred embodiments, the modafinil compound is modafinil.

As used herein, "bioavailable" is intended to mean a portion of the administered dose that is absorbed in the blood stream and can readily be determined by techniques known in the art, such as, for example, by measuring the blood serum level of a compound.

As used herein, the term "pharmaceutically acceptable" refers to those compounds, materials, compositions, and/or dosage forms which are, within the scope of sound medical judgment, suitable for contact with the tissues of human beings and animals without excessive toxicity, irrigation, allergic response, or other problem complications commensurate with a reasonable benefit/risk ratio.

As used herein, the term "subject" refers to a warm blooded animal such as a mammal, preferably a human or a human child, which is afflicted with, or has the potential to be afflicted with one or more diseases and conditions described herein.

As used herein, "therapeutically effective amount" refers to an amount which is effective in reducing, eliminating, treating, preventing or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

As used herein, "unit dose" means a single dose which is capable of being administered to a subject, and which can be readily handled and packaged, remaining as a physically and chemically stable unit dose comprising either a modafinil compound, or a pharmaceutically acceptable composition comprising a modafinil compound.

As used herein, a "lower alkyl alcohol" refers to a branched or straight-chained alkyl alcohol containing from 1 to 6 carbon atoms, such as methanol, ethanol, n-propanol, isopropanol, n-butanol, isobutyl alcohol, sec-butyl alcohol, t-butyl alcohol, pentanol, hexanol; with preferred lower alkyl alcohols including ethanol, propanol and isopropanol.

As used herein, the term "arylalkyl alcohol" refers to aryl-substituted C₁-C₆ alkyl alcohols such as benzyl alcohol, phenethyl alcohol, diphenylmethyl alcohol (benzhydrol); with preferred arylalkyl alcohols including benzyl alcohol, α-phenethyl alcohol and β-phenethyl alcohol.

As used herein, "antioxidant" is intended to indicate any substance useful to retard deterioration by oxidation or to inhibit reactions promoted by oxygen or peroxides.

As used herein, "lipid" is intended to indicate a fat, oil, wax, sterol, glycerol ether, triglyceride, or combination thereof.

As used herein, the term "about" refers to a range of values ± 10% of a specified value. For example, the phrase "about 50%" includes ± 10% of 50, or from 45 to 55%.

In certain preferred embodiments, the compositions comprise a modafinil compound at a concentration of 1 to 500 mg/ml. In certain more preferred embodiments, a modafinil compound is present from 1 to 200 mg/ml, and most preferably from 20 to 80 mg/ml. The compositions of the present invention comprise at least one surfactant. In other preferred embodiments, there are three surfactants, and other more preferred embodiments include one or two surfactants. In certain embodiments, the surfactant acts as the primary solubilizing agent. In other embodiments, the compositions comprise from 0.5% to 50% or from 1% to 20% total surfactant. The amount of total surfactant is more preferably at least 5%, and less than about 40%, depending upon the surfactant and the additional components of the composition. Preferably, appropriate surfactants are those, when admixed with a modafinil compound, result in particle-forming compositions and compositions of particles, and more preferably, in stable suspensions. One skilled in the art can readily determine the appropriate surfactant or combination of surfactants, and their relative amounts, by use of conventional techniques and observing the characteristics of the resultant composition. Several factors can be considered, including for example, the solubility of the modafinil compound in the solution, the degree of precipitation of the modafinil compound, the degree of solubilization or emulsification of the solution and the stability of the solution over a period of time.

The surfactants include polyoxyethylene sorbitan fatty acid esters, polyethylene glycol ethers, saturated polyglycolized glycerides, fatty acid esters of polyethylene glycols, medium chain monoglycerides, medium chain fatty acid esters, d-α-tocopheryl polyethylene glycol succinate, polyethylene/propylene glycol copolymers, block copolymers of ethylene oxide and propylene oxide, polyoxyl stearates, ethoxylated castor oils, and ethoxylated hydroxystearic acids. Additional surfactants can be found in The Handbook of Pharmaceutical Excipients, 2nd Ed., (The Pharmaceutical Press, London and American Pharmaceutical Association (1994)), a common text in the field.

The polyoxyethylene sorbitan fatty acid esters (polysorbates) are non-ionic surfactants (detergents) that may comprise a mixture of fatty acids. Commercially available examples are polyoxyethylene (20) sorbitan monolaurate (such as Tween^{®} 20), polyoxyethylene (40) sorbitan monopalmitate (such as Tween^{®} 40), polyoxyethylene (80) sorbitan monooleate (such as Tween^{®} 80) and sorbitan monolaurate (such as Span^{®} 20). Preferred polyoxyethylene sorbitan fatty acid esters are polyoxyethylene (80) sorbitan monooleate (in particular, Tween^{®} 80) and sorbitan monolaurate (in particular, Span^{®} 20). The saturated polyglycolized glycerides include, for example, mono-, di-, or triglycerides. The di-fatty acid esters of polyethylene glycols include, for example, Gelucire^{®} 44/14 (primarily a fatty acid ester of PEG-1500, available from Gattefossé, Saint-Priest, France). The medium chain monoglycerides, wherein the chain length is from 6 to 10 carbon atoms, include for example, glyceryl monocaprylate (Imwitor^{®} 308), glyceryl monocaproate (Capmul^{®} MCM C-8), glyceryl caprylate/caprate (Capmul^{®} MCM) and a mixture of polyoxyethylene glyceryl caprylate and polyoxyethylene glyceryl caproate (Labrasol^{®}). The medium chain fatty acid esters include medium chain length triglycerides, such as a mixture of glyceryl tricaprate and glyceryl tricaprilate (Miglyol^{®} 612). The block copolymers of ethylene oxide and propylene oxide include, for example, polyoxyethylene-polyoxypropylene block co-polymer (Pluronic^{®} F-68). The polyoxyl stearates include polyethoxylated (40) stearic acid (Myrj^{®} 52). The ethoxylated castor oils include, for example, polyethoxylated (60) hydrogenated castor oil (Cremophor^{®} EL). The ethoxylated hydroxystearic acids include, for example; polyethylene glycol 660 hydroxystearate (Solutol^{®} HS 15). Some-surfactants are-solid or semisolid at room temperature, e.g., glyceryl monocaprylate, and Gelucire^{®} 44/14.

Examples of surfactants which are particularly effective as the primary solubilizing agent, such as those compositions where the surfactant comprises more than 50% of the composition, include polyethoxylated (60) hydrogenated castor oil (such as Cremophor^{®} EL), polyethylene glycol 660 hydroxystearate (such as Solutol^{®}-HS 15), polyethoxylated (40) stearic acid (such as Myrj^{®} 52) and polyoxyethylene (80) sorbitan monooleate (such as Tween^{®} 80).

In other preferred embodiments, the compositions comprise more than one surfactant. In certain embodiments, the additional surfactants may be selected from any,of the aforementioned surfactants. Preferably, an additional, or second, surfactant, is a polyoxyethylene sorbitan fatty acid ester, and more preferably is polyoxyethylene (80) sorbitan monooleate (in particular, Tween^{®} 80) and sorbitan monolaurate (in particular, Span^{®} 20).

In other embodiments, the compositions comprise a polyoxyethylene sorbitan fatty acid ester, preferably polyoxyethylene (80) sorbitan monooleate (in particular, Tween^{®} 80); medium chain monoglycerides, in particular, glyceryl caprylate/caprate(Capmul^{®} MCM); and medium chain length triglycerides, such as a mixture of glyceryl tricaprate and glyceryl tricaprilate (in particular, Miglyol^{®} 612). A preferred composition comprises Tween^{®} 80, Capmul^{®} MCM, and Miglyol^{®} 612.

According to the invention, the composition comprise at least one organic solvent. In certain preferred embodiments, there are three solvents, and other more preferred embodiments include one or two solvents. In certain preferred embodiments, the amounts of any additional solvents comprise from about 0.5% to about 50% (v/v) of the composition, with a more preferred amount of about 1% to about 50% (v/v), and a most preferred amount about 5% to about 20% (v/v).

Preferably, an appropriate organic solvent is one which increases the solubility of a modafinil compound in a particle-forming composition and does not adversely impact upon the formation of suspended particles.

In certain preferred embodiments, the compositions comprise at least one organic solvent including glycerin, propylene glycol, diethylene glycol ethyl ether, propylene carbonate, tetraglycol (also known as glycofurol), medium chain length monoglycerides, or polyethyleneglycols. Medium chain length monoglycerides include glyceryl monocaprylate (Imwitor^{®}), glyceryl caprylate/caprate (such as Capmul^{®}) and polyoxyethylene glyceryl caproate (such as Labrasol^{®}). Preferred organic solvents include polyethylene glycols or "PEG", which refer to a liquid or solid polymer of the general formula H(OCH₂)CH₂)ₙOH, wherein n is at least 4. The preferred PEG has an average molecular weight of from about 200 to about 1500, and commercially available PEG materials include PEG-200, PEG-300, PEG-400, PEG-540, PEG-600, PEG-800, PEG-1000 and PEG-1450. All are commercially available from, for example, from Union Carbide Corporation in both food or pharmaceutical grades. Preferred PEG solvents for use in the present composition include PEG-300, PEG-400 and PEG 1450, with PEG-300 and PEG-400 being more preferred.

In certain embodiments, the compositions comprise glycol and a surfactant. Preferably the surfactant is an ethoxylated hydroxystearic acid, and in particular is polyethylene glycol 660 hydroxystearate. In certain preferred embodiments, the ratio of glycol to surfactant is 1:1.

In other preferred embodiments, the compositions comprise an additional, or second solvent, which is preferably a lower alkyl alcohol or an alkylaryl alcohol, and more preferably benzyl alcohol, α-phenethyl alcohol or β-phenethyl alcohol. In more preferred embodiments, the solvent system includes mixtures of a polyethylene glycol and an arylalkyl alcohol. More preferred embodiments include mixtures of PEG-400 and benzyl alcohol, PEG-400 and α-phenethyl alcohol, and PEG-400 and β-phenethyl alcohol. A most preferred embodiment includes a mixture of 95:5 (v/v) PEG-400:benzyl alcohol.

In certain preferred embodiments, the compositions comprise a modafinil compound, or preferably modafinil, at a concentration of 1 to 100 mg/ml, preferably from 1 to 60 mg/ml and more preferably from 20 to 50 mg/ml; a first surfactant selected from a polyoxyethylene sorbitan fatty acid ester, a polyethylene glycol ether, a saturated polyglycolized glyceride, a fatty acid ester of a polyethylene glycol, a medium chain monoglyceride, a medium chain fatty acid ester, d-α-tocopheryl polyethylene glycol succinate, a polyethylene/propylene glycol copolymer, block copolymers of ethylene oxide and propylene oxide, a polyoxyl stearate, an ethoxylated castor oil, and an ethoxylated hydroxystearic acid; and may additionally comprise a second surfactant selected from a polyoxyethylene sorbitan fatty acid ester; and may further additionally comprise an organic solvent selected from glycerin, propylene glycol, diethylene glycol ethyl ether, propylene carbonate, a medium chain length monoglyceride, and a polyethyleneglycol. In a more preferred embodiment, the compositions are pharmaceutically acceptable.

In certain further preferred embodiments, the first surfactant is a saturated polyglycolized glyceride, a fatty acid ester of a polyethylene glycol, or a medium chain monoglyceride; the second surfactant is a polyoxyethylene sorbitan fatty acid ester; and the organic solvent is a polyethyleneglycol. In more preferred embodiments, the first surfactant is glyceryl caprylate/caprate, glyceryl monocaprylate or polyethoxylated (40) stearic acid; the second surfactant is sorbitan monolaurate; and the organic solvent is PEG-300 or PEG-400.

In certain most preferred embodiments, the compositions comprise 90% PEG-400, 5% sorbitan monolaurate, 5% glyceryl caprylate/caprate (w/w/w), or in particular, 90% PEG-400, 5% Span^{®} 20, 5% Capmul^{®} MCM (w/w/w). In other most preferred embodiments, the compositions comprise 90% PEG-400, 5% sorbitan monolaurate, 5% glyceryl monocaprylate (w/w/w), or in particular, 90% PEG-400, 5% Span® 20, 5% Imwitor^{®} 308 (w/w/w). In another most preferred embodiment, the compositions comprise 90% PEG-400, 5% sorbitan monolaurate, 5% polyethoxylated (40) stearic acid (w/w/w), or in particular, 90% PEG-400, 5% Span^{®} 20, 5% Myrj^{®} 52 (w/w/w).

In other more preferred embodiments, the first surfactant is glyceryl caprylate/caprate, glyceryl monocaprylate, polyethoxylated (40) stearic acid or a mixture of polyoxyethylene glyceryl caprylate and polyoxyethylene glyceryl caproate; the second surfactant is polyoxyethylene (80) sorbitan monooleate; and the organic solvent is PEG-300 or PEG-400.

In other most preferred embodiments, the compositions comprise 70% PEG-400, 15% polyoxyethylene (80) sorbitan monooleate, 15% glyceryl caprylate/caprate (w/w/w), in particular, 70% PEQ-400, 15% Tween^{®} 80, 15% Capmul^{®} MCM (w/w/w). In another most preferred embodiment, the compositions comprise 70% PEG-400, 55% polyoxyethylene (80) sorbitan monooleate, 15% glyceryl monocaprylate (w/w/w), in particular, 70% PEG-400, 15% Tween^{®} 80, 15% Imwitor^{®} 308 (w/w/w). In a further most preferred embodiment, the compositions comprise 70% PEG-400.15% polyoxyethylene (80) sorbitan monooleate, 15% polyethoxylated (40) stearic acid (w/w/w), in particular, 70% PEG-400,15% Tween^{®} 80, 15% Myrj^{®} 52 (w/w/w). In an additional most preferred embodiment, the compositions comprise 70% PEG-400, 15% polyoxyethylene (80) sorbitan monooleate, 15% of a mixture of polyoxyethylene glyceryl caprylate and polyoxyethylene glyceryl caproate (w/w/w), in particular, 70% PEG-400, 15% Tween^{®} 80, 15% Labrasol^{®} (w/w/w).

In another embodiment, the present invention provides for a method of preparing a composition of particles, wherein the particles comprise a modafinil compounds, comprising contacting a particle-forming composition of the invention with an aqueous medium. Preferably, the modafinil compound is modafinil.

In a preferred embodiment, the amount of surfactant is from 1% to 50% by weight of the composition. In a preferred embodiment, the modafinil compound is modafinil. In another preferred embodiment, the composition of particles is formed by contacting the particle-forming composition with the aqueous medium in vitro.

In another embodiment, the present invention provides for the use of a modafinil compound, or preferably modafinil for the preparation of pharmaceutical compositions useful In the treatment of a disease or disorder.

In certain preferred embodiments, the pharmaceutical compositions are useful for treatment of sleepiness, such as excessive daytime sleepiness associated with narcolepsy, or sleepiness associated with sleep apneas, tiredness, Parkinson's disease, cerebral ischemia, stroke, sleep apneas, eating disorders, attention deficit hyperactivity disorder, cognitive dysfunction or fatigue, such as fatigue resulting from multiple sclerosis ("MS fatigue"); and for promotion of wakefulness, stimulation of appetite, or stimulation of weight gain.

In certain embodiments, administration of a therapeutically effective amount of the composition can be readily determined by the attending diagnostician, as one skilled in the art, by the use of conventional techniques and by observing results ' obtained under analogous circumstances. In determining the therapeutically effective amount, a number of factors are considered by the attending diagnostician, including the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

A therapeutically effective amount of a modafinil compound will vary depending upon a number of factors, including the dosage of the drug to be administered, the chemical characteristics (e.g., hydrophobicity) of the compounds employed, the potency of the compounds, the type of disease, the diseased state of the patient, and the route of administration. Generally, treatment is initiated with small dosages, which can then be increased by small increments until the optimum desired effect under the circumstances is achieved.

In certain preferred embodiments, the compositions comprise one or more unit doses of a modafinil compound. In certain more preferred embodiments, the compositions comprise one unit dose of modafinil. Preferable daily doses of modafinil range from about 0.01 to 100 mg/kg of body weight. By way of general guidance, daily doses for humans range from about 0.1 mg to about 2000 mg. Preferably the unit dose range is from about 1 to about 500 mg administered one to four times a day, and even more preferably from about 10 mg to about 400 mg, one to two times a day. In certain preferred embodiments, the unit dose is 100 or 200 mg. In other preferred embodiments, a unit dose is one that is necessary to achieve a blood serum level of about 0.05 to 30 µg/ml, and more preferably, of about 1 to about 20 µg/ml in a subject.

Upon administration of either of the compositions to a subject, the modafinil compound has a blood serum level of about 0.05 to about 30 µg/ml in said subject. In a preferred embodiment, the modafinil compound has a blood serum level of about 1 to about 20 µg/ml in said subject. In another preferred embodiment, the composition being administered to achieve the desired blood serum levels is a particle-forming composition comprising a modafinil compound. In a further preferred embodiment, the composition being administered to achieve the desired blood serum levels is an aqueous composition of particles, wherein the particles comprise a modafinil compound. In more preferred embodiments, the modafinil compound is modafinil.

In a further embodiment, the present invention provides for compositions that are suitable for oral administration to a subject. Oral administration includes ingestion in the form of a liquid composition, such as a syrup, elixir, emulsion; or a capsule. A preferred embodiment is in the form of a capsule, and more preferably as hard capsules, comprising gelatin, hydroxypropylmethylcellulose ("cellulose"), or starch. Another more preferred embodiment is in the form of soft gelatin capsules. In particular, the soft gel capsules comprise a non-aqueous composition. In additional preferred embodiments, the composition being used for oral administration is a particle-forming composition comprising a modafinil compound. In further preferred embodiment, the composition being used for oral administration is an aqueous composition of particles, wherein the particles comprise a modafinil compound.

In other embodiments of the invention, the compositions may also be prepared in admixture with additional pharmaceutically-acceptable excipients or components to further promote effective therapeutic use. The excipients may include lipids, for example, those which are useful to change particle size; antibacterial agents such as benzyl alcohol or methyl paraben; antioxidants such as ascorbic acid, sodium bisulfite, and fatty acid esters of ascorbic acid, such as ascorbyl palmitate; chelating agents such as ethylene diaminetetraacetic acid; buffers such as acetates, citrates or phosphates and agents for the adjustment of toxicity such as sodium chloride or dextrose; and other excipients such as flavorings, sweetening agents and coloring agents. Other appropriate excipients can readily be determined by one skilled in the art, and may further include those found in The Handbook of Pharmaceutical Excipients, 2nd Ed., (The Pharmaceutical Press, London and American Pharmaceutical Association (1994)).

The compositions of the present invention comprise modafinil compounds, which may be readily prepared by one skilled in the art using conventional methods. Methods for preparing modafinil and various derivatives appear in U.S. Pat. No. 4,177,290, and methods for preparing other modafinil compounds appear in U.S. Pat. No. 4,927,855, 5,719,168 and in U.S. Patent Application No. 60/204,789.

There is wide latitude in formulation of the compositions of the present invention. The compositions of particles may be formed by contacting the particle-forming compositions comprising a modafinil compound with an aqueous medium in vitro, i.e., subjected to predilution, prior to ingestion by the subject, or in vivo, e.g. contact with aqueous contents of the gastrointestinal composition of the subject. If the composition is prediluted, a preferable dilution ratio is from about 1:1000 (1 part formulation to 999 parts aqueous medium) to about 1:2 (1 part formulation to 1 part aqueous medium). More preferably, the dilution ratio is from about 1:500 (1 part formulation to 499 parts aqueous medium) to about 1:3 (1 part formulation to 2 parts aqueous medium). By way of general guidance, for administration to humans a convenient ratio is about 1: 250; which is a rough correspondence to a 1 ml unit dose dispersed in an 8-ounce glass of an aqueous liquid.

In certain preferred embodiments, when the particle-forming composition is contacted with an aqueous medium a homogeneous, stable composition comprising suspended particles is formed. Preferably, the particles comprise a modafinil compound. Topically, the particles are thermodynamically stable, and are formed spontaneously upon mixing, without external mechanical agitation. The particles are preferably in the microparticles size range with a diameter of 1 to 1000 nm. More preferably; the particles have a diameter of 1 to 400 nm; and most preferably 1 to 100 nm.

A feature of these preferred compositions is that they are translucent and optically isotropic. A useful indication of the particle size is the degree of optical transparency of a given volume of water comprising a given amount of formulation. This is due to the scattering of visible light by the particles, with the larger particles causing greater scattering. In general, the greater the optical transparency, the smaller the particle size. High optical transparency, i.e., bluish haze invisible or nearly invisible, generally indicates a particle size of less than 100 nm. A distinct bluish haze generally indicates, a particle size from 100 nm to 400 nm. Without intending to be bound by theory, it is noted that particle size tends to be essentially constant for a given formulation, regardless of the dilution ratio. If particles fail to form, an increase in dilution ratio, or an adjustment of the amount and type of surfactant may be used to promote particle formation.

An additional feature of these preferred compositions is that they remain physically stable, which allows for desirable and effective use of the compositions as pharmaceutically acceptable formulations. An indication of a stable compositions is retention of the same outward appearance and properties over an extended period of time, sufficient to retain pharmaceutical acceptability. In stable compositions, the particles generally remain intact and sufficiently dispersed or suspended in the liquid medium. Typically, creaming or sedimentation is minimal, or otherwise, the particles can be redispersed upon mild agitation. Additionally, the particles do not readily or irreversibly aggregate, coalesce, or otherwise revert back to two separate bulk phases.

The compositions of the present invention may be a liquid, semi-solid, or solid at room temperature. If liquid, the compositions may be contained in a capsule. If semi-solid or solid, the compositions can be in the form of a capsule or tablet.

Whether a composition according to the invention is a liquid, semi-solid, or solid at room temperature, may depend upon the selection of components, or other concerns such as commercial viability and administration. For example, a semi-solid or solid formulation is convenient for manufacturing unit doses of modafinil compound in the form of a capsule, including both hard gelatin and soft gelatin capsules, and tablets. When the liquid or solid formulation contacts an aqueous medium, e.g., gastrointestinal liquids, the formulation disperses into suspended particles in which modafinil compound is biologically available.

Compositions whose inert or non-active components (i.e., components other than modafinil) are all liquid at room temperature can be prepared by simply mixing the components without heating. The desired amount of a modafinil compound can be weighed out and dissolved in the mixture of inert components, without heating. Moderate heating, preferably less than 60° C, can be applied to hasten complete mixing of the inert components, to hasten dissolution of a modafinil compound, or both.

Preparation of compositions comprising one or more components that are solid at room temperature is carried out at a moderately elevated temperature, preferably less than 60° C. While moderate heating can be useful, excessive heating can cause decomposition of one or more components of the formulation. For example, decomposition of Polysorbate 80 can occur at temperatures above 60° C. Decomposition of Polysorbate 80 may occur if maintained at 90°C for more than one hour. As will be appreciated by one of ordinary skill in the art, any deleterious effects of heat accumulate with time. Therefore, when heat is applied, time and temperature will typically be balanced against one another.

The materials, methods, and examples presented herein are intended to be illustrative. Unless otherwise defined, all technical and scientific terms are intended to have their art-recognized meanings.

### Examples

### A. Materials:

All the materials in the following examples are commercially available or can be readily prepared by one skilled in the art by known or readily available literature methods. The surfactants were used as supplied with no additional purification or dilution. Solvents of USP/NF grade or better were employed.

### B. Methods:

### 1. HPLC

The following HPLC method was used to measure modafinil content in the compositions: A 10mL serum bottle or 4mL screw cap vial containing the surfactant solution saturated with modafinil was filtered through a 1.2 µm syringe filter as indicated in the sample preparations described hereinafter. 10µL of the clear solution was diluted to 1mL with 990µL of dimethylsulfoxide (Fischer Certified ACS grade). 10µL of the diluted solution was used for each injection in the HPLC analysis for modafinil content in each mixture. The column conditions are listed below.
Flow rate: 1.2mL/min.
Column: ODS, 4.6 x 20mm, Column Temp: 30 °C
Mobil phase: 80%(65% Acetonitrile/35%1M phosphate buffer) 20% water
Analysis time: 5 minutes
Wavelength: 222 nanometers

Concentration was calculated by comparison to area from a modafinil standard used at 0.4mg/mL with appropriate dilution. The results are shown in Example 8, Table 1. Each measurement of concentration was an average of two injections.

### 2. H₂O Dispersion

To determine if a formulation would be suitable as a SEDDS, a 1:20 dilution of each test formulation was prepared with water and timed for the formation of a cloudiness or appearance of a precipitate. In most cases, the failure of the SEEDS was noted by observing a coarse emulsion (as evidenced by cloudiness) or obvious solid particle precipitation within about 10 minutes of mixing.

### 3. Method for Measurements of Blood Level in Rats Given Modafinil Formulations

21 adult male Sprague-Dawley rats (body weight: 359±6 grams) were fasted overnight prior to use. Each oral formulation was administered via oral gavage (n=3/formulation). The dose of modafinil administered was 100 mg/kg using a dose volume of 3.3 ml/kg. Blood was collected from the lateral tail vein at 0.25, 0.5, 1, 2, 4 and 6 hours post dose. The blood was collected on wet ice and spun at 13,000RPM for 10 minutes. The supernatant (plasma) was collected and frozen on dry ice. Samples were stored at -70 °C until analysis. The blood serum levels of modafinil in these experiments were measured by LC/MS, as shown in Example 9, Table 2.

### Example 1: Preparation of 90% PEG 400, 5% Span^{®} 20.5% Capmul^{®} MCM (w:w:w)

To 90 grams of PEG 400 were added 5 grams of Span^{®} 20 and 5 grams of Capmul^{®} MCM with stirring until the solution was homogeneous. To a separate container were added 0.1 gram of modafinil and 1 mL of the mixed solvent/surfactant with stirring and heating to 55-60° C. The solution was allowed to cool to room temperature and any undissolved solid was removed by filtering the solution using a 1.2µL syringe filter.

### Example 2: Preparation of 90% PEG 400, 5% Span^{®} 20,5% Imwitor^{®} 308 (w:w:w)

A quantity of solid Imwitor^{®} 308 was melted and 5 grams were added to 90 grams of PEG-400 and 5 grams of Span^{®} 20 with stirring until the solution was homogenous. To a separate container were added 0.1 gram of modafinil and 1 mL of the mixed solvent/surfactant with stirring and heating to 55-60° C. The solution was allowed to cool to room temperature. Since this mixture is semi-solid, gentle warming to about 35-40° C was necessary before viscosity was low enough to allow filtration to remove any undissolved modafinil by filtering the solution using a 1.2µL syringe filter.

### Example 3: Preparation of 90% PEG 400,5% Span^{®} 20,5% Myrj^{®}-52 (w:w:w)

A quantity of solid Myrj^{®}-52 was melted and 5 grams were added to 90 grams of PEG-400 and 5 grams of Span^{®} 20 with stirring until the solution was homogenous. To a separate container were added 0.1 gram of modafinil and 1 mL of the mixed solvent/surfactant with stirring and heating to 55-60° C. The solution was allowed to cool to room temperature. Since this mixture is semi-solid, gentle warming to about 35-40 °C was necessary before viscosity was low enough to allow filtration to remove any undissolved modafinil by filtering the solution using a 1.2µL syringe filter.

### Example 4: Preparation of 70% PEG 400, 15% Tween^{®} 80, 15% Labrasol^{®} (w:w:w)

To 70 grams of PEG-400 were added 15 grams of Tween^{®} 80 (Polysorbate 80) and 15 grams of Labrasol^{®} with stirring until the solution was homogeneous. To a separate container were added 0.1 gram of modafinil was weighed and 1 mL of the mixed solvent/surfactant with stirring and heating to 55-60° C. The solution was allowed to cool to room temperature and any undissolved solid was removed by filtering the solution using a 1.2µL syringe filter.

### Example 5: Preparation of 70% PEG 400, 15% Tween^{®} 80,15% Myrj^{®}-52 (w:w:w)

A quantity of solid Myrj^{®}-52 was melted and 15 grams were added to 70 grams of PEG-400 and 15 grams of Tween^{®} 80 with stirring until the solution was homogenous. To a separate container were added 0.1 gram of modafinil and 1 mL of the mixed solvent/surfactant with stirring and heating to 55-60° C. The solution was allowed to cool to room temperature. Since this mixture is semi-solid, gentle warming to about 35-40° C was necessary before viscosity was low enough to allow filtration to remove any undissolved modafinil by filtering the solution using a 1.2µL syringe filter.

### Example 6: Preparation of 70% PEG 400,15% Tween^{®} 80, 15% Capmul^{®} MCM (w:w:w)

To 70 grams of PEG-400 were added 15 grams of Tween^{®} 80 (Polysorbate 80) and 15 grams of Capmul^{®} MCM with stirring until the solution was homogeneous. To a separate container were added 0.1 gram of modafinil and 1 mL of the mixed solvent/surfactant with stirring and heating to 55-60° C. The solution was allowed to cool to room temperature and any undissolved solid was removed by filtering the solution using a 1.2µL syringe filter.

### Example 7: Preparation of 70% PEG 400,15% Tween^{®} 80,15% Imwitor^{®} 308 (w:w:w)

A quantity of solid Imwitor^{®}-308 was melted and 15 grams were added to 70 grams of PEG-400 and 15 grams of Tween^{®} 80 with stirring until the solution was homogenous. To a separate container were added 0.1 gram of modafinil and 1 mL of the mixed solvent/surfactant with stirring and heating to 55-60°C. The solution was allowed to cool to room temperature. Since this mixture is semi-solid, gentle warming to about 35-40 °C was necessary before viscosity was low enough to allow filtration to remove any undissolved modafinil by filtering the solution using a 1.2µL syringe filter.

### Example 8: Solubility of Modafinil in Particle-forming Compositions

The solubility of modafinil in the compositions of Examples 1-7, as measured by HPLC, is shown below in Table 1.

**Table 1:**

| **Solubility of Modafinil in Particle-forming Compositions** | | | | |
|---|---|---|---|---|
| Excipients (w:w:w) | | | | Solubility (mg/ml) |
| Ex. 1 | 90% PEG 400 | 5% Span 20 | 5% Capmul^{®} MCM | 50 |
| Ex. 2 | 90% PEG 400 | 5% Span 20 | 5% Imwitor^{®} 308 | 52 |
| Ex. 3 | 90% PEG 400 | 5% Span 20 | 5% Myrj^{®} 52 | 57 |
| Ex. 4 | 70% PEG 400 | 15% Tween^{®} 80 | 15% Labrasol^{®} | 43 |
| Ex. 5 | 70% PEG 400 | 15% Tween^{®} 80 | 15% Myrj^{®} 52 | 47 |
| Ex. 6 | 70% PEG 400 | 15% Tween^{®} 80 | 15% Capmul^{®} MCM | 40 |
| Ex. 7 | 70% PEG 400 | 15% Tween^{®} 80 | 15% Imwitor^{®} 308 | 44 |

### Example 9: Blood Serum Levels of Modafinil in Rats

The blood serum levels of modafinil in rats, upon administration of compositions of Examples 1-6, is shown below in Table 2. The Oraplus^{®} composition is intended to mimic the bioavailability of solid modafinil dosed in an oral fashion such as a tablet, but without the difficulty of administering a tablet to the rat. Oraplus^{®} is an oral suspending vehicle that is commercially available (Paddock Laboratories, Minneapolis, MN), and is primarily composed of purified water, microcrystalline cellulose, sodium carboxymethylcellulose, xanthan gum, carrageenan, citric acid and sodium phosphate (as buffers), simethicone (antifoaming agent), and potassium sorbate and methyl paraben (preservatives).

**Table 2:**

| **Blood Serum Levels of Modafinil in Rats** | | | | | | | |
|---|---|---|---|---|---|---|---|
| | **BLOOD SERUM LEVEL (ng/ml)** | | | | | | |
| **Modafinil Solutions** | **Ex. 1** | **Ex.2** | **Ex. 3** | **Ex. 4** | **Ex. 5** | **Ex. 6** | **Oraplus** |
| **TIME (Hrs.)** | | | | | | | |
| 0.25 | 2.6 | 11.2 | 2.6 | 2.2 | 0.5 | 1.0 | 3.4 |
| 0.5 | 2.2 | 5.1 | 4.2 | 2.3 | 9.7 | 3.4 | 4.9 |
| 1 | 2.3 | 16.5 | 3.8 | 1.4 | 8.2 | 1.7 | 3.0 |
| 2 | 1.2 | 1.7 | 2.8 | 0.6 | 5.8 | 3.4 | 1.9 |
| 4 | 0.6 | 1.4 | 0.7 | 0.6 | 3.5 | 1.9 | 0.4 |
| 6 | 0.4 | 0.4 | 0.2 | 0.3 | 0.2 | 0.4 | 0.2 |

## Claims

1. A particle-forming composition comprising a modafinil compound, at least one surfactant, and an organic solvent, **characterized in that** the composition spontaneously forms a stable suspension of non-crystalline particles comprising the modafinil compound when contacted with an aqueous medium, wherein the non-crystalline particles have a diameter of 1 to 1,000 nm.

2. An aqueous composition of non-crystalline particles comprising at least one surfactant, an organic solvent, and a modafinil compound, wherein the non-crystalline particles have a diameter of 1 to 1,000 nm, and wherein the composition is liquid, homogeneous, stable, translucent, and optically isotropic.

3. The composition of claims 1 or 2, wherein the composition is pharmaceutically acceptable.

4. The composition of claims 1 or 2, wherein the surfactant or surfactants comprise from 0.5% to 50% (w/w) of the composition.

5. The composition of claim 4, wherein the surfactant or surfactants comprise from 1% to 20% (w/w) of the composition.

6. The composition of claims 1 or 2, wherein the surfactant or surfactants is a polyoxyethylene sorbitan fatty acid ester, a polyethylene glycol ether, a saturated polyglycolized glyceride, a fatty acid ester of polyethylene glycol, a medium chain monoglyceride, a medium chain fatty acid ester, d-α-tocopheryl polyethylene glycol succinate, a polyethylene/propylene glycol copolymer, block copolymers of ethylene oxide and propylene oxide, a polyoxyl stearate, an ethoxylated castor oil, or an ethoxylated hydroxystearic acid.

7. The composition of claim 6, comprising a second surfactant.

8. The composition of claim 7, wherein the second surfactant is a polyoxyethylene sorbitan fatty acid ester.

9. The composition of claim 8, wherein the second surfactant is sorbitan monolaurate or Polysorbate 80.

10. The composition of claim 6, wherein the composition comprises Polysorbate 80, glyceryl caprylate/caprate and a mixture of glyceryl tricaprate and glyceryl tricaprilate.

11. The composition of claims 1 or 2, wherein the organic solvent is glycerin, propylene glycol, diethylene glycol ethyl ether, propylene carbonate, a medium chain length monoglyceride, or a polyethyleneglycol.

12. The composition of claim 11, further comprising benzyl alcohol, α-phenethyl alcohols or β-phenethyl alcohol.

13. The composition of claims 1 or 2, comprising one or more unit doses of a modafinil compound.

14. The composition of claim 13, comprising one unit dose of a modafinil compound.

15. The composition of claim 14, wherein the unit dose is 200 mg.

16. The composition of claim 14, wherein the unit dose is 100 mg.

17. The composition of claim 1 or claim 2, wherein the composition is suitable for oral administration to a subject.

18. The composition of claim 17, wherein the composition is encapsulated within a capsule.

19. The composition of claim 18, wherein the capsule is a soft gelatin capsule.

20. The composition of claim 18, wherein the capsule is a hard capsule.

21. The composition of claim 17, wherein the composition is a syrup, elixir, or emulsion.

22. The composition of any of the preceding claims, wherein the modafinil compound is modafinil.

23. The composition of claim 22, wherein modafinil is present at a concentration of 1 to 500 mg/ml.

24. The composition of claim 23, wherein modafinil is present at a concentration of 1 to 200 mg/ml.

25. The composition of claims 1 or 2, wherein a modafinil compound is present at a concentration of 1 to 100 mg/ml a first surfactant selected from a polyoxyethylene sorbitan fatty acid ester, a polyethylene glycol ether, a saturated polyglycolized glyceride, a fatty acid ester of a polyethylene glycol, a medium chain monoglyceride, a medium chain fatty acid ester, d-α-tocopheryl polyethylene glycol succinate, a polyethylene/propylene glycol copolymer, block copolymers of ethylene oxide and propylene oxide, a polyoxyl stearate, an ethoxylated castor oil, and an ethoxylated hydroxystearic acid; a second surfactant selected from a polyoxyethylene sorbitan fatty acid ester; and an organic solvent selected from glycerin, propylene glycol, diethylene glycol ethyl ether, propylene carbonate, a medium chain length monoglyceride, or a polyethylene glycol.

26. The composition of claim 25, wherein the modafinil compound is modafinil.

27. The composition of claim 26, wherein the first surfactant is a saturated polyglycolized glyceride, a fatty acid ester of a polyethylene glycol, or a medium chain monoglyceride; the second surfactant is a polyoxyethylene sorbitan fatty acid ester; and the organic solvent is a polyethyleneglycol.

28. The composition of claim 27, wherein the first surfactant is glyceryl caprylate/caprate, glyceryl monocaprylate or polyethoxylated (40) stearic acid; the second surfactant is sorbitan monolaurate; and the organic solvent is PEG-300 or PEG-400.

29. The composition of claim 28, wherein the composition comprises 90% PEG-400, 5% sorbitan monolaurate, 5% glyceryl caprylate/caprate (w/w/w).

30. The composition of claim 28, wherein the composition comprises 90% PEG-400, 5% sorbitan monolaurate, 5% glyceryl monocaprylate (w/w/w).

31. The composition of claim 28, wherein the composition comprises 90% PEG-400, 5% sorbitan monolaurate, 5% polyethoxylated (40) stearic acid (w/w/w).

32. The composition of claim 27, wherein the first surfactant is glyceryl caprylate/caprate, glyceryl monocaprylate, polyethoxylated (40) stearic acid or a mixture of polyoxyethylene glyceryl caprylate and polyoxyethylene glyceryl caproate; the second surfactant is polyoxyethylene (80) sorbitan monooleate; and the organic solvent is PEG-300 or PEG400.

33. The composition of claim 32, wherein the composition comprises 70%PEG-400, 15% polyoxyethylene (80) sorbitan monooleate, 15% glyceryl caprylate/caprate (w/w/w).

34. The composition of claim 32, wherein the composition comprises 70% PEG-400, 15% polyoxyethylene (80) sorbitan monooleate, 15% glyceryl monocaprylate (w/w/w).

35. The composition of claim 32, wherein the composition comprises 70% PEG-400, 15% polyoxyethylene (80) sorbitan monooleate, 15% polyethoxylated (40) stearic acid (w/w/w).

36. The composition of claim 32, wherein the composition comprises 70% PEG-400, 15% polyoxyethylene (80) sorbitan monooleate, 15% of a mixture of polyoxyethylene glyceryl caprylate and polyoxyethylene glyceryl caproate (w/w/w).

37. The composition of any preceding claim wherein the modafinil compound is (-)benzhydrylsulfinyl-acetamide,

38. A composition according to any of claims 1 to 37 for use as a medicament.

39. A method of preparing a composition of particles, comprising contacting a particle forming composition of claim 1 with an aqueous medium.

40. The method of claims 39, wherein the particle forming composition is contacted with an aqueous medium in vitro.

41. The method of claim 39, wherein the modafinil compound is modafinil.

42. The use of the particle-forming composition of claim 1 in the manufacture of a medicament for the treatment of a disease or disorder in a subject.

43. The use of the composition of non-crystalline particles of claim 2 in the manufacture of a medicament for the treatment of a disease or disorder in a subject.

44. The use of claim 42 or claim 43, wherein the modafinil compound is modafinil.

45. The use of claim 42 or claim 43, wherein the treatment is of sleepiness, tiredness, Parkinson's disease, cerebral ischemia, stroke, sleep apneas, eating disorders, attention deficit hyperactivity disorder, cognitive dysfunction or fatigue; and for the promotion of wakefulness, stimulation of appetite, or stimulation of weight gain to a patient in need thereof.

46. The use according to any of claims 42 to 45, wherein the modafinil compound is (-)benzhydrylsulfinyl-acetamide.

47. The composition of claims 1 or 2, wherein the non-crystalline particles have a diameter of 1 to 400 nm.

48. The composition of claims 1 or 2, wherein the non-crystalline particles have a diameter of 1 to 100 nm.

## Patentansprüche

1. Teilchen-bildende Zusammensetzung, umfassend eine Modafinil-Verbindung, mindestens ein grenzflächenaktives Mittel und ein organisches Lösungsmittel, **dadurch gekennzeichnet, daß** die Zusammensetzung spontan eine stabile Suspension von nicht-kristallinen Teilchen, umfassend die Modafinil-Verbindung, wenn in Kontakt mit einem wäßrigen Medium, bildet, wobei die nicht-kristallinen Teilchen einen Durchmesser von 1 bis 1,000 nm aufweisen.

2. Wäßrige Zusammensetzung von nicht-kristallinen Teilchen, umfassend mindestens ein grenzflächenaktives Mittel, ein organisches Lösungsmittel und eine Modafinil-Verbindung, wobei die nicht-kristallinen Teilchen einen Durchmesser von 1 bis 1.000 nm aufweisen, und wobei die Zusammensetzung flüssig, homogen, stabil, transluzent und optisch isotrop ist.

3. Zusammensetzung gemäß den Ansprüchen 1 oder 2, wobei die Zusammensetzung pharmazeutisch verträglich ist.

4. Zusammensetzung gemäß den Ansprüchen 1 oder 2, wobei das grenzflächenaktive Mittel oder die grenzflächenaktiven Mittel von 0,5% bis 50% (Gew./Gew.) der Zusammensetzung umfassen.

5. Zusammensetzung gemäß Anspruch 4, wobei das grenzflächenaktive Mittel oder die grenzflächenaktiven Mittel von 1% bis 20% (Gew./Gew.) der Zusammensetzung umfassen.

6. Zusammensetzung gemäß den Ansprüchen 1 oder 2, wobei das grenzflächenaktive Mittel oder die grenzflächenaktiven Mittel ein Polyoxyethylensorbitfettsäureester, ein Polyethylenglykolether, ein gesättigtes polyglykolisiertes Glycerid, ein Fettsäureester von Polyethylenglykol, ein Monoglycerid mittlerer Kette, ein Fettsäureester mittlerer Kette, d-α-Tocopherylpolyethylenglykolsuccinat, ein Polyethylen/Propylenglykolcopolymer, Blockcopolymere von Ethylenoxid und Propylenoxid, ein Polyoxylstearat, ein ethoxyliertes Castoröl oder eine ethoxylierte Hydroxystearinsäure ist.

7. Zusammensetzung gemäß Anspruch 6, umfassend ein zweites grenzflächenaktives Mittel.

8. Zusammensetzung gemäß Anspruch 7, wobei das zweite grenzflächenaktive Mittel ein Polyoxyethylensorbitfettsäureester ist.

9. Zusammensetzung gemäß Anspruch 8, wobei das zweite grenzflächenaktive Mittel Sorbitmonolaurat oder Polysorbat 80 ist.

10. Zusammensetzung gemäß Anspruch 6, wobei die Zusammensetzung Polysorbat 80, Glycerylcaprylat/caprinat und ein Gemisch von Glyceryltricaprinat und Glyceryltricaprylat umfaßt.

11. Zusammensetzung gemäß den Ansprüchen 1 oder 2, wobei das organische Lösungsmittel Glycerin, Propylenglykol, Diethylenglykolethylether, Propylencarbonat, ein Monoglycerid mittlerer Kettenlänge oder ein Polyethylenglykol ist.

12. Zusammensetzung gemäß Anspruch 11, weiter umfassend Benzylalkohol, α-Phenethylalkohol oder β-Phenethylalkohol.

13. Zusammensetzung gemäß den Ansprüchen 1 oder 2, umfassend ein oder mehrere Dosiseinheiten einer Modafinil-Verbindung.

14. Zusammensetzung gemäß Anspruch 13, umfassend eine Dosiseinheit einer Modafinil-Verbindung.

15. Zusammensetzung gemäß Anspruch 14, wobei die Dosiseinheit 200 mg ist.

16. Zusammensetzung gemäß Anspruch 14, wobei die Dosiseinheit 100 mg ist.

17. Zusammensetzung gemäß Anspruch 1 oder Anspruch 2, wobei die Zusammensetzung zur oralen Verabreichung einem Subjekt geeignet ist.

18. Zusammensetzung gemäß Anspruch 17, wobei die Zusammensetzung in einer Kapsel eingekapselt ist.

19. Zusammensetzung gemäß Anspruch 18, wobei die Kapsel eine weiche Gelatinekapsel ist.

20. Zusammensetzung gemäß Anspruch 18, wobei die Kapsel eine Hartkapsel ist.

21. Zusammensetzung gemäß Anspruch 17, wobei die Zusammensetzung ein Sirup, ein Elixier oder eine Emulsion ist.

22. Zusammensetzung gemäß einem der vorhergehenden Ansprüche, wobei die Modafinil-Verbindung Modafinil ist.

23. Zusammensetzung gemäß Anspruch 22, wobei Modafinil in einer Konzentration von 1 bis 500 mg/ml vorliegt.

24. Zusammensetzung gemäß Anspruch 23, wobei Modafinil in einer Konzentration von 1 bis 200 mg/ml vorliegt.

25. Zusammensetzung gemäß den Ansprüchen 1 oder 2, wobei eine Modafinil-Verbindung in einer Konzentration von 1 bis 100 mg/ml, ein erstes grenzflächenaktives Mittel, ausgewählt aus einem Polyoxyethylensorbitfettsäureester, einem Polyethylenglykolether, einem gesättigten polyglykolisierten Glycerid, einem Fettsäureester von einem Polyethylenglykol, einem Monoglycerid mittlerer Kette, einem Fettsäureester mittlerer Kette, d-α-Tocopherylpolyethylenglykolsuccinat, einem Polyethylen/Propylenglykolcopolymer, Blockcopolymeren von Ethylenoxid und Propylenoxid, einem Polyoxylstearat, einem ethoxyliertesn Castoröl und einer ethoxylierten Hydroxystearinsäure, ein zweites grenzflächenaktives Mittel, ausgewählt aus einem Polyoxyethylensorbitfettsäureester, und ein organisches Lösungsmittel, ausgewählt aus Glycerin, Propylenglykol, Diethylenglykolethylether, Propylencarbonat, einem Monoglycerid mittlerer Kettenlänge oder einem Polyethylenglykol, vorliegt.

26. Zusammensetzung gemäß Anspruch 25, wobei die Modafinil-Verbindung Modafinil ist.

27. Zusammensetzung gemäß Anspruch 26, wobei das erste grenzflächenaktive Mittel ein gesättigtes polyglykolisiertes Glycerid, ein Fettsäureester eines Polyethylenglykols oder ein Monoglycerid mittlerer Kettenlänge ist, das zweite grenzflächenaktive Mittel ein Polyoxyethylensorbitfettsäureester ist, und das organische Lösungsmittel ein Polyethylenglykol ist.

28. Zusammensetzung gemäß Anspruch 27, wobei das erste grenzflächenaktive Mittel Glycerylcaprylat/caprinat, Glycerylmonocaprylat oder polyethoxylierte(40) Stearinsäure ist, das zweite grenzflächenaktive Mittel Sorbitmonolaurat ist, und das organische Lösungsmittel PEG-300 oder PEG-400 ist.

29. Zusammensetzung gemäß Anspruch 28, wobei die Zusammensetzung 90% PEG-400, 5% Sorbitmonolaurat, 5% Glycerylcaprylat/caprinat (Gew./Gew./Gew.) umfaßt.

30. Zusammensetzung gemäß Anspruch 28, wobei die Zusammensetzung 90% PEG-400, 5% Sorbitanmonolaurat, 5% Glycerylmonocaprylat (Gew./Gew./Gew.) umfaßt.

31. Zusammensetzung gemäß Anspruch 28, wobei die Zusammensetzung 90% PEG-400, 5% Sorbitmonolaurat, 5% polyethoxylierte(40) Stearinsäure (Gew./Gew./Gew.) umfaßt.

32. Zusammensetzung gemäß Anspruch 27, wobei das erste grenzflächenaktive Mittel Glycerylcaprylat/caprinat, Glycerylmonocaprylat, polyethoxylierte(40) Stearinsäure oder ein Gemisch von Polyoxyethylenglycerylcaprylat und Polyoxyethylenglycerylcapronat ist, das zweite grenzflächenaktive Mittel Polyoxyethylen(80)-sorbitmonooleat ist, und das organische Lösungsmittel PEG-300 oder PEG-400 ist.

33. Zusammensetzung gemäß Anspruch 32, wobei die Zusammensetzung 70% PEG-400, 15% Polyoxyethylen(80)-sorbitmonooleat, 15% Glycerylcaprylat/caprinat (Gew./Gew./Gew.) umfaßt.

34. Zusammensetzung gemäß Anspruch 32, wobei die Zusammensetzung 70% PEG-400, 15% Polyoxyethylen(80)-sorbitmonooleat, 15% Glycerylmonocaprylat (Gew./Gew./Gew.) umfaßt.

35. Zusammensetzung gemäß Anspruch 32, wobei die Zusammensetzung 70% PEG-400, 15% Polyoxyethylen(80)-sorbitmonooleat, 15% polyethoxylierte(40) Stearinsäure (Gew./Gew./Gew.) umfaßt.

36. Zusammensetzung gemäß Anspruch 32, wobei die Zusammensetzung 70% PEG-400, 15 % Polyoxyethylen(80)-sorbitmonooleat, 15% eines Gemisches von Polyoxyethylenglycerylcaprylat und Polyoxyethylenglycerylcapronat (Gew./Gew./Gew.) umfaßt.

37. Zusammensetzung gemäß einem vorhergehenden Anspruch, wobei die Modafinil-Verbindung (-)Benzhydrylsulfinylacetamid ist.

38. Zusammensetzung gemäß einem der Ansprüche 1 bis 37 zur Verwendung als ein Medikament.

39. Verfahren zur Herstellung einer Zusammensetzung von Teilchen, umfassend das Inkontaktbringen einer Teilchen-bildenden Zusammensetzung gemäß Anspruch 1 mit einem wäßrigen Medium.

40. Verfahren gemäß Anspruch 39, wobei die Teilchen-bildende Zusammensetzung mit einem wäßrigen Medium *in vitro* in Kontakt gebracht wird.

41. Verfahren gemäß Anspruch 39, wobei die Modafinil-Verbindung Modafinil ist.

42. Verwendung der Teilchen-bildenden Zusammensetzung gemäß Anspruch 1 zur Herstellung eines Medikaments zur Behandlung einer Krankheit oder Störung in einem Subjekt.

43. Verwendung der Zusammensetzung von nicht-kristallinen Teilchen gemäß Anspruch 2 zur Herstellung eines Medikaments zur Behandlung einer Krankheit oder Störung in einem Subjekt.

44. Verwendung gemäß Anspruch 42 oder Anspruch 43, wobei die Modafinil-Verbindung Modafinil ist.

45. Verwendung gemäß Anspruch 42 oder Anspruch 43, wobei die Behandlung bezüglich Schlaflosigkeit, Ermüdung, Parkinson-Krankheit, zerebraler Ischämie, Schlaganfall, Schlaf-Atemstillständen, Eßstörungen, Aufmerksamkeitsdefizit-Hyperaktivitätsstörung, kognitiver Dysfunktion oder Erschöpfung, oder zur Förderung von Wachsamkeit, Appetitstimulation oder Gewichtszunahmestimulation ist.

46. Verwendung gemäß einem der Ansprüche 42 bis 45, wobei die Modafinil-Verbindung (-)Benzhydrylsulfinylacetamid ist.

47. Zusammensetzung gemäß den Ansprüchen 1 oder 2, wobei die nicht-kristallinen Teilchen einen Durchmesser von 1 bis 400 nm aufweisen.

48. Zusammensetzung gemäß den Ansprüchen 1 oder 2, wobei die nicht-kristallinen Teilchen einen Durchmesser von 1 bis 100 nm aufweisen.

## Revendications

1. Composition formant des particules comprenant un composé de modafinil, au moins un tensioactif, et un solvant organique, **caractérisée en ce que** la composition forme spontanément une suspension stable de particules non cristallines comprenant le composé de modafinil lorsqu'elle est mise en contact avec un milieu aqueux, dans laquelle les particules non cristallines ont un diamètre de 1 à 1 000 nm.

2. Composition aqueuse de particules non cristallines comprenant au moins un tensioactif, un solvant organique, et un composé de modafinil, dans laquelle les particules non cristallines ont un diamètre de 1 à 1 000 nm, et dans laquelle la composition est liquide, homogène, stable, translucide, et optiquement isotropique.

3. Composition selon les revendications 1 ou 2, dans laquelle la composition est pharmaceutiquement acceptable.

4. Composition selon les revendications 1 ou 2, dans laquelle le ou les tensioactifs représentent de 0,5 % à 50 % (m/m) de la composition.

5. Composition selon la revendication 4, dans laquelle le ou les tensioactifs représentent de 1 % à 20 % (m/m) de la composition.

6. Composition selon les revendications 1 ou 2, dans laquelle le ou les tensioactifs sont un ester d'acide gras de polyoxyéthylène sorbitane, un éther de polyéthylène glycol, un glycéride polyglycolisé saturé, un ester d'acide gras de polyéthylène glycol, un monoglycéride à chaîne moyenne, un ester d'acide gras à chaîne moyenne, le succinate de d-α-tocophéryl polyéthylène glycol, un copolymère polyéthylène/propylène glycol, des copolymères séquencés d'oxyde d'éthylène et d'oxyde de propylène, un stéarate polyoxyéthylénique, une huile de ricin éthoxylée, ou un acide hydroxystéarique éthoxylé.

7. Composition selon la revendication 6, comprenant un second tensioactif.

8. Composition selon la revendication 7, dans laquelle le second tensioactif est un ester d'acide gras de polyoxyéthylène sorbitane.

9. Composition selon la revendication 8, dans laquelle le second tensioactif est le monolaurate de sorbitane ou le polysorbate 80.

10. Composition selon la revendication 6, dans laquelle la composition comprend du polysorbate 80, du caprylate/caprate de glycéryle et un mélange de tricaprate de glycéryle et de tricaprilate de glycéryle.

11. Composition selon les revendications 1 ou 2, dans laquelle le solvant organique est la glycérine, le propylène glycol, l'éther éthylique de diéthylène glycol, le carbonate de propylène, un monoglycéride à longueur de chaîne moyenne, ou un polyéthylène glycol.

12. Composition selon la revendication 11, comprenant en outre de l'alcool benzylique, de l'alcool α-phénéthylique ou de l'alcool β-phénéthylique.

13. Composition selon les revendications 1 ou 2, comprenant une ou plusieurs doses unitaires d'un composé de modafinil.

14. Composition selon la revendication 13, comprenant une dose unitaire d'un composé de modafinil.

15. Composition selon la revendication 14, dans laquelle la dose unitaire est de 200 mg.

16. Composition selon la revendication 14, dans laquelle la dose unitaire est de 100 mg.

17. Composition selon la revendication 1 ou la revendication 2, dans laquelle la composition convient à une administration orale à un sujet.

18. Composition selon la revendication 17, dans laquelle la composition est encapsulée dans une capsule.

19. Composition selon la revendication 18, dans laquelle la capsule est une capsule molle.

20. Composition selon la revendication 18, dans laquelle la capsule est une capsule dure.

21. Composition selon la revendication 17, dans laquelle la composition est un sirop, un élixir, ou une émulsion.

22. Composition selon l'une quelconque des revendications précédentes, dans laquelle le composé de modafinil est le modafinil.

23. Composition selon la revendication 22, dans laquelle le modafinil est présent à une concentration de 1 à 500 mg/ml.

24. Composition selon la revendication 23, dans laquelle le modafinil est présent à une concentration de 1 à 200 mg/ml.

25. Composition selon les revendications 1 ou 2, dans laquelle un composé de modafinil est présent à une concentration de 1 à 100 mg/ml, un premier tensioactif choisi parmi un ester d'acide gras de polyoxyéthylène sorbitane, un éther de polyéthylène glycol, un glycéride polyglycolisé saturé, un ester d'acide gras d'un polyéthylène glycol, un monoglycéride à chaîne moyenne, un ester d'acide gras à chaîne moyenne, le succinate de d-α-tocophéryl polyéthylène glycol, un copolymère polyéthylène/propylène glycol, des copolymères séquencés d'oxyde d'éthylène et d'oxyde de propylène, un stéarate polyoxyéthylénique, une huile de ricin éthoxylée, et un acide hydroxystéarique éthoxylé ; un second tensioactif choisi parmi un ester d'acide gras de polyoxyéthylène sorbitane ; et un solvant organique choisi parmi la glycérine, le propylène glycol, l'éther éthylique de diéthylène glycol, le carbonate de propylène, un monoglycéride à longueur de chaîne moyenne, ou un polyéthylène glycol.

26. Composition selon la revendication 25, dans laquelle le composé de modafinil est le modafinil.

27. Composition selon la revendication 26, dans laquelle le premier tensioactif est un glycéride polyglycolisé saturé, un ester d'acide gras d'un polyéthylène glycol, ou un monoglycéride à chaîne moyenne ; le second tensioactif est un ester d'acide gras de polyoxyéthylène sorbitane ; et le solvant organique est un polyéthylène glycol.

28. Composition selon la revendication 27, dans laquelle le premier tensioactif est le caprylate/caprate de glycéryle, le monocaprylate de glycéryle ou l'acide stéarique polyéthoxylé (40) ; le second tensioactif est le monolaurate de sorbitane ; et le solvant organique est le PEG-300 ou le PEG-400.

29. Composition selon la revendication 28, dans laquelle la composition comprend 90 % de PEG-400, 5 % de monolaurate de sorbitane, 5 % de caprylate/caprate de glycéryle (m/m/m).

30. Composition selon la revendication 28, dans laquelle la composition comprend 90 % de PEG-400, 5 % de monolaurate de sorbitane, 5 % de monocaprylate de glycéryle (m/m/m).

31. Composition selon la revendication 28, dans laquelle la composition comprend 90 % de PEG-400, 5 % de monolaurate de sorbitane, 5 % d'acide stéarique polyéthoxylé (40) (m/m/m).

32. Composition selon la revendication 27, dans laquelle le premier tensioactif est le caprylate/caprate de glycéryle, le monocaprylate de glycéryle, l'acide stéarique polyéthoxylé (40) ou un mélange de caprylate de polyoxyéthylène glycéryle et de caproate de polyoxyéthylène glycéryle ; le second tensioactif est le monooléate de polyoxyéthylène (80) sorbitane ; et le solvant organique est le PEG-300 ou le PEG-400.

33. Composition selon la revendication 32, dans laquelle la composition comprend 70 % de PEG-400, 15 % de monooléate de polyoxyéthylène (80) sorbitane, 15 % de caprylate/caprate de glycéryle (m/m/m),

34. Composition selon la revendication 32, dans laquelle la composition comprend 70 % de PEG-400, 15 % de monooléate de polyoxyéthylène (80) sorbitane, 15 % de monocaprylate de glycéryle (m/m/m).

35. Composition selon la revendication 32, dans laquelle la composition comprend 70 % de PEG-400, 15 % de monooléate de polyoxyéthylène (80) sorbitane, 15 % d'acide stéarique polyéthoxylé (40) (m/m/m),

36. Composition selon la revendication 32, dans laquelle la composition comprend 70 % de PEG-400, 15 % de monooléate de polyoxyéthylène (80) sorbitane, 15 % d'un mélange de caprylate de polyoxyéthylène glycéryle et de caproate de polyoxyéthylène glycéryle (m/m/m).

37. Composition selon l'une quelconque des revendications précédentes, dans laquelle le composé de modafinil est le (-)benzhydrylsulfinyl-acétamide.

38. Composition selon l'une quelconque des revendications 1 à 37, destinée à une utilisation en tant que médicament.

39. Procédé de préparation d'une composition de particules, comprenant la mise en contact d'une composition formant des particules selon la revendication 1 avec un milieu aqueux.

40. Procédé selon la revendication 39, dans lequel la composition formant des particules est mise en contact avec un milieu aqueux in vitro.

41. Procédé selon la revendication 39, dans lequel le composé de modafinil est le modafinil.

42. Utilisation de la composition formant des particules selon la revendication 1 dans la fabrication d'un médicament destiné au traitement d'une maladie ou d'un trouble chez un sujet.

43. Utilisation de la composition de particules non cristallines selon la revendication 2 dans la fabrication d'un médicament destiné au traitement d'une maladie ou d'un trouble chez un sujet.

44. Utilisation selon la revendication 42 ou la revendication 43, dans laquelle le composé de modafinil est le modafinil.

45. Utilisation selon la revendication 42 ou la revendication 43, dans laquelle le traitement est destiné à la somnolence, la fatigue, la maladie de Parkinson, l'ischémie cérébrale, l'accident vasculaire cérébral, les apnées du sommeil, les troubles de l'alimentation, le trouble déficitaire de l'attention avec hyperactivité, la fatigue ou le dysfonctionnement cognitif ; et à favoriser la veille, stimuler l'appétit, ou stimuler la prise de poids chez un patient en ayant besoin.

46. Utilisation selon l'une quelconque des revendications 42 à 45, dans laquelle le composé de modafinil est le (-)benzhydrylsulfinylacétamide.

47. Composition selon les revendications 1 ou 2, dans laquelle les particules non cristallines ont un diamètre de 1 à 400 nm.

48. Composition selon les revendications 1 ou 2, dans laquelle les particules non cristallines ont un diamètre de 1 à 100 nm.
